(19) Europäisches Patentamt
European Patent Office
Office européen des brevets

(11) Publication number: **0 002 426**
**B1**

(12) # EUROPEAN PATENT SPECIFICATION

(45) Date of publication of patent specification: **07.10.81**

(51) Int. Cl.$^3$: **A 61 K 7/32**

(21) Application number: **78850022.1**

(22) Date of filing: **01.12.78**

(54) **Antiperspirant.**

(30) Priority: **01.12.77 SE 7713617**

(43) Date of publication of application:
**13.06.79 Bulletin 79/12**

(45) Publication of the grant of the European patent:
**07.10.81 Bulletin 81/40**

(84) Designated Contracting States:
**BE CH DE FR GB IT LU NL SE**

(56) References cited:

**FR - M - 7919**
**FR - A - 2 013 248**
**FR - A - 2 150 613**
**US - A - 2 614 963**
**US - A - 3 079 299**

(73) Proprietor: **Playtex-Wallco Aktiebolag**
**Box 209**
**S-791 01 Falun (SE)**

(72) Inventor: **Broberg, Berndt Fredrik Julius**
**Byvägen 48**
**S-151 52 Södertälje (SE)**
Inventor: **Evers, Hans Christer Arvid**
**Hovslagarvägen 17**
**S-151 59 Södertälje (SE)**
Inventor: **Juhlin, Lennart August Albert**
**Döbelnsgatan 30 H**
**S-752 37 Uppsala (SE)**

(74) Representative: **Wurm, Bengt Runio et al,**
**Patent and Trade Mark Department Ab Astra**
**S-151 85 Södertälje (SE)**

**EP 0 002 426 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Antiperspirant

The present invention relates to an antiperspirant composition.

The object of the present invention is to obtain an antiperspirant composition which gives a real blockade of the function of the sweat glands preferably in the axillae.

In most commercial perspirant inhibiting cosmetics present on the market the active perspirant inhibiting component is usually an astringent salt of aluminium, zink, zirconium or a rare earth metal. Such salts are as a rule not immediately active as antiperspirants after the first treatment but require a number of treatments during a time period until a desired degree of antiperspiration effect can be obtained. Having these problems in mind investigations have been carried out in order to find compounds which have the rapid antiperspiration effect without requiring a number of treatments in order to obtain useful antiperspiration effect, which compounds are also innocouos to clothes and skin. Various anticholinergic compounds, which prevent the stimulation of the ecrine sweat-glands by inhibiting the action of acetylcholine, have also been used.

Further it is previously known as disclosed in Swedish Patent Specification S/N 7200316—3 (publ. no. 381 567) to use 3-phenylcarbo(β-diethylamino)-ethoxy-2,3-dihydrobenzofuran hydrochloride, which is known to possess analgesic, hypotensive, local anesthetic, and antispasmodic effects, as an active component in antiperspirants in amounts of 0.01—2.0% of an aqueous solution.

Chemical Abstracts *71* (1969), 48238b, discloses that procaine when administered electrophoretically possesses an antihydrotic effect. However, procaine administered without electrophoresis but only topically will not give any antihydrotic effect.

Remington's Pharmaceutical Sciences, 15th Ed., 1975, Mack Publishing Comp. Easton, Pennsylvania, USA, p. 986—996 discloses that local anesthetics can be used for topical application on ulcers, wounds, and mucous surfaces, whereby different local anesthetics can be used in concentrations from 0.5% to 20%. It is also known from this document that mixtures of two drugs do not enhance the anesthesia nor prolong the duration of action. The document, however, does not disclose the action of the local anesthetics on intact skin, and particularly not in preventing excessive perspiration.

According to the present invention more significant results have been obtained using a mixture of local anesthetics as active component compared with a single local anesthetic as active component.

In Merck Index 9th Ed., 1976, Merck & Co., Rahway, N.J., USA, pages 135, 136, 193, 427, 507, 508, 1227 and 1244 a number of different local anesthetics is disclosed i.a. for topical application. The document does not disclose the use of local anesthetics as antiperspirants, nor a combination of local anesthetic for said purpose.

It is further known, from US Patent Specification 2.614.963, to prepare compositions containing a local anesthetic, whereby the compositions are intended for keratinic exfoliation purposes.

French Patent Specification 2.013.248 relates to antihemorrhoidal compositions which may contain a local anesthetic apparently to give relief to hurting hemorrhoids. It is also known from this document to obtain emulsions containing a water soluble salt of a local anesthetic.

French Patent Specification 2.150.613 relates to a composition to be used to inhibit ejaculation for certain reasons. Such compositions, which are active on mucous membranes comprise dibucaine, a biologically acceptable carrier and a fluoro-chloro hydrocarbon.

It has now surprisingly been found that it is possible to obtain a rapid and completely effective antiperspiration effect by means of the present invention, which is characterized in that the antiperspirant composition contains as active component a mixture of a first local anesthetic compound which is prilocaine, tetracaine, butanilicaine or trimecaine in base form, and a second local anesthetic compound which is benzocaine, lidocaine, bupivacaine, mepivacaine, dibucaine, etidocaine, tetracaine, butanilicaine, or trimecaine in base form.

These mixtures give oils which can be applied easily on a carrier, which then is applied in the axilla.

Preferred suitable mixtures are prilocaine and benzocaine in the weight ratio 65:35 to 80:20, preferably 70:30;

prilocaine and lidocaine in the weight ratio 42:58 to 80:20, suitably 47:53 to 62:38, preferably 55:45;

prilocaine and etidocaine in the weight ratio 55:45 to 95:5, suitably 60:40 to 80:20, preferably 70:30;

prilocaine and mepivacaine in the weight ratio 80:20 to 97:3, suitably 85:15 to 90:10, preferably 87:13;

prilocaine and bupivacaine in the weight ratio of 72:28 to 97:3, suitably 78:22 to 88:12, preferably 85:15.

An antiperspirant composition according to the present invention is usually administered as an emulsion containing the anesthetic active agent, an emulsifier, and/or thickening agent and water and when necessary a solvent for the agent.

As suitable emulsifiers Tween® 80, Arlaton® 289, and lectine can be used.

Arlaton® 289 is a polyoxyethylene fatty acid ester, and Tween® 80 is a sorbitanefatty acid ester.

As suitable thickening agent Carbopol®, Metocel®, Pluronic® and carboxymethylcellulose can be used.

In certain cases the thickening agent may also have emulsifying properties. Thus Carbopol® and Pluronic® can be used both as thickening agent and emulsifier.

As suitable solvents for the active substance alcohols having 1—6 carbon atoms, and 1—5 hydroxy groups (ethanol, propanol, isopropanol, glycerol, sorbitol, ethylene glycol, propylene glycol;) polyalkylene glycols, e.g. polyethylene glycols (molecular weights of 300 to 10 000); monoglycerides, wherein the fatty acid component has 12—18 carbon atoms, e.g. glycerol monolaurate, and other materials in order to improve different properties of the cosmetic preparations. Bactericides, e.g. hexachlorophene, fungicides and antibiotics, e.g. neomycine, can be incorporated as well.

An antiperspirant composition can be prepared within the following limits for components present.

| | | |
|---|---|---|
| Local anesthetically active substance | 0.5—100% | by weight |
| Thickening agent | 0—30% | by weight |
| Emulsifier | 0—99.4% | by weight |
| Water | 0—20% | by weight |

Above given compounds, except for benzocaine, dibucaine, and tetracaine are represented by the general formulae

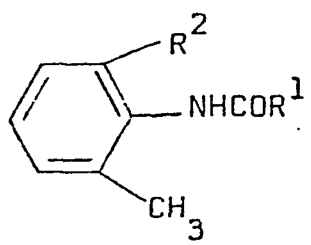 wherein $R^1$ is 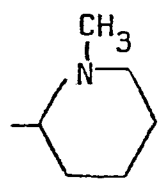 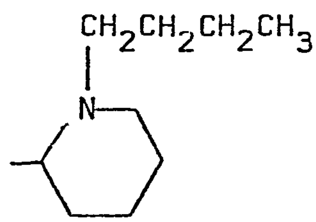

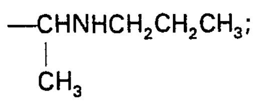; 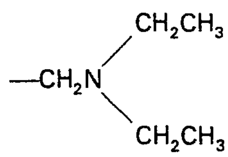 ; or 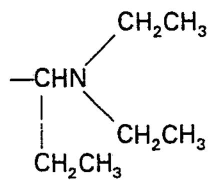

and $R^2$ is hydrogen or methyl, whereby $R^2$ is hydrogen when $R^1$ is 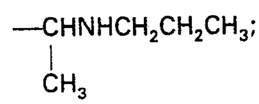;

or whereby $R^1$ is $-CH_2NH(CH_2)_3CH_3$ and $R^2$ is chloro, or wherein $R^1$ is $-CH_2N(C_2H_5)_2$, $R^2$ is methyl and a further methyl group is present in para position to the carbamoyl containing side chain.

Benzocaine, 4-aminobenzoic acid ethylester, has the formula

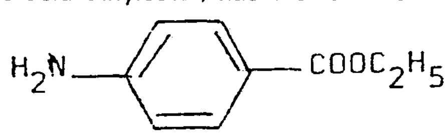

Tetracaine, 2-(dimethylamino)ethylester of p-butylamino benzoic acid has the formula

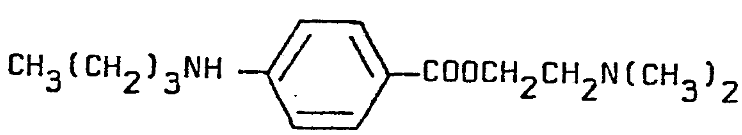

Dibucaine, 2-butoxy-N-(2-diethylaminoethyl)-cinchoniamide has the formula

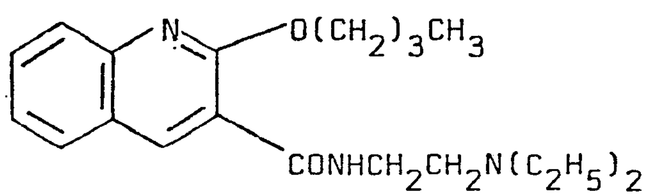

1-methyl-2-(2',6'-xylylcarbamoyl)piperidine is known under the generic name mepivacaine and is sold under the trade mark Carbocaine®.

1-butyl-2-(2',6'-xylylcarbamoyl)piperidine is known under the generic name bupivacaine and is sold under the trade mark Marcaine®.

2-propylamino-N-(2-tolyl)propionamide is known under the generic name prilocaine and is sold under the trade mark Citanest®.

Diethylaminoacet-2',6'-xylidide is known under the generic name lidocaine and is sold under the trade mark Xylocaine®.

2-(ethylpropylamino)-2',6'-n-butyroxylidide is known under the generic name etidocaine and is sold under the trade mark Duranest®.

2-butylamino-6'-chloro-o-aceto-toluide is known under the generic name butanilicaine.

2-diethylamino-2',4',6'-trimethylacetanilide is known under the generic name trimecaine.

The amount of the compound of the above formula active against perspiration and present in the antiperspirant compositions is usually 0.5—100% by weight, preferably 0.5—10%, and more preferably 2—5%. Higher concentrations than these will usually not be proportionally more active than average or lower concentrations within the ranges given. When lower concentrations than the lower amounts in the ranges given are used the amount of preparation necessary to be applied in order to obtain an amount large enough of the active compound will then become so large that it is unpractical.

The amounts of the compositions given which are applied at one occasion are generally such that 1 to 50 milligrams of active compound, e.g. lidocaine-prilocaine mixture in base form are deposited, on the place or places, usually in the axillae, to be treated. Preferably 1 to 30 mg, more preferably 5 to 20 mg, are applied. The use on other parts of the body is usually adopted so that the amount of active compound, which is applied in an amount within above given ranges, is proportional to the surface.

The aqueous, water and alcohol containing or other compositions of the invention in the forms of solutions, emulsions, creames, ointments, or in any other form are applied onto the skin normally at ambient temperature, preferably by means of roll-on applicator or with applicator or manual rubbing in. Spraying of a composition may also be used.

The body place treated is usually the axillae, but also the chest, the back, the seat, the arms, the palms of the hands, the face, the forehead, the genital regions, the thighs and the feet can be treated.

The invention gives several significative advantages and provides for the production of antiperspirant compositions which are superior to the compositions now on the market in several respects. The very small quantities of material needed to be applied are not felt to be sticky, heavy or desiccating on the skin.

For the determination of the reduction of sweat secretion, several methods can be used. At small quantities a solution containing 5% orthophthaldialdehyde in xylol is used, which is painted onto the place in question. If sweat perspires in the openings of the sweat-glands these become black-coloured.

When an antiperspirant according to Example 1 is applied in one axilla of a person and placebo (emulsion without local anesthetically active compound) is applied in the second axilla of the same person a definite difference is established between treated and "untreated" axilla using this method. The number of black sweat-gland openings were definitely reduced in the treated axilla of all test persons, whereby treated axillae gave a white impression and untreated axillae gave a black impression.

Biological effect

The antiperspiration effect of the present invention was determined in an experimental investigation of dermal analgesia and influence upon adrenaline-induced local sweating by epidermal application of different local anesthetic formulations.

In the investigation it was shown in 12 volunteers that the sweat response, induced by intracutaneous administration of adrenaline, was partly blocked by epidermal application of 1) a combination of prilocaine and lidocaine, 2) lidocaine alone, and 3) prilocaine alone. Both the sweat response and dermal analgesia (tested by pin-pricking) was most pronounced with the formulation consisting of an eutectic mixture of lidocaine base and prilocaine base, in a total concentration of 10%, as an aqueous emulsion. The other two tested formulations, containing 10% lidocaine and 10% prilocaine base respectively, were less effective, both with regard to sweat response inhibition and to dermal analgesia, even though these produced demonstrable significant effects, over that of a placebo emulsion.

Material and method

12 healthy medical students took part in the study.

Pre-formed pads, consisting of cellulose fibres, 2 times 2 centimetres, were soaked by a standardized procedure in either an emulsion containing 5% lidocaine base + 5% prilocaine base, an emulsifying agent and water, 10% lidocaine base in the same vehicle, 10% prilocaine base in the same vehicle, or a placebo emulsion containing the emulsifying agent and water only. On one forearm of the volunteer the four different emulsions were applied under occlusive dressings consisting of an impervious tape, while on the other arm the four emulsions were applied under a non-occlusive

dressing, using surgical tape (3M). A certain distance between the application areas was chosen in order to avoid any risk of interference between them.

On the arms of the volunteers 11 and 12 the emulsions were applied directly to the skin, with no cellulose pads, but with occlusive and non-occlusive dressings applied as in the other volunteers.

The application time for the pads was 60 minutes.

Immediately after removal of the pads, the square areas under the two types of dressing were marked at the edges. A line was also marked within each applied area dividing them into two identical triangles. One of these surfaces were then used by pin-prick experiments, the other for studying the sweat response.

*Testing of dermal analgesia*

The pin-pricking procedure was carried out with disposable dental needles. In each of the test areas 10 pin-pricks were made. 10/10 represents full analgesia, 0/10 no analgesia.

*Testing of sweat response*

Just under the apex of each test area triangle an intradermal wheal 0.1 ml adrenaline 1:10 000 was injected. Pain on injection was noted. The test area was then swabbed with OPT (5% orthophthaldialdehyde in xylol). After drying the OPT-applied area a plastic ring with adhesive on the back side was placed over the visible vasoconstricted area caused by the adrenaline effect, within the test triangle previously covered by the test compositions. The number of black-coloured sweat pores within the standardized diameter hole in the plastic rings was counted under a direct vision microscope.

Results

*Sweat response*

The sweat response was most effectively blocked in the lidocaine-prilocaine areas: By the student's t-test analysis it was found that:

| | | |
|---|---|---|
| Lidocaine-prilocaine versus placebo: | p=0.001 | Occlusive bandage |
| Lidocaine-prilocaine versus lidocaine: | p=0.01 | |
| Lidocaine-prilocaine versus prilocaine: | p=0.05 | |
| Lidocaine-prilocaine versus placebo: | p=0.01 | Non-occlusive bandage |
| Lidocaine-prilocaine versus lodocaine: | p=0.05 | |
| Lidocaine-prilocaine versus prilocaine: | p=0.05 | |

whereby p is the statistical probability value obtained calculating the statistical significance.

The lidocaine and prilocaine emulsions were also found to be significantly more effective than placebo as evident from Table I below under occlusive bandage and be more effective than placebo under non-occlusive bandage.

TABLE I

OPT-test

| Patient No | Occlusion L—P | Occlusion L | Occlusion P | Occlusion Placebo | Non-Occlusion L—P | Non-Occlusion L | Non-Occlusion P | Non-Occlusion Placebo |
|---|---|---|---|---|---|---|---|---|
| 1 | 16 | 16 | 17 | 48 | 25 | 27 | 26 | 26 |
| 2 | 16 | 28 | 52 | 54 | 19 | 25 | 25 | 40 |
| 3 | 15 | 24 | 18 | 48 | 10 | 24 | 21 | 48 |
| 4 | 9 | 16 | 22 | 36 | 25 | 24 | 26 | 54 |
| 5 | 9 | 20 | 15 | 20 | 20 | 19 | 18 | 24 |
| 6 | 16 | 22 | 28 | 22 | 25 | 32 | 40 | 36 |
| 7 | 15 | 22 | — | 36 | 12 | 42 | 46 | 28 |
| 8 | 20 | 25 | 24 | 42 | 18 | 24 | 18 | 28 |
| 9 | 22 | 20 | 30 | 34 | 20 | 29 | 34 | 48 |
| 10 | 18 | 24 | 24 | 36 | 15 | 24 | 24 | 22 |
| $M_{1-10}$ | 15.6 | 21.7 | 25.6 | 37.6 | 18.9 | 27.0 | 27.8 | 35.4 |
| 11 | 15 | 12 | 24 | 30 | 20 | 22 | 14 | 12 |
| 12 | 37 | 26 | 16 | 46 | 13 | 33 | 20 | 32 |
| $M_{1-12}$ | 17.33 | 21.25 | 24.54 | 37.66 | 18.5 | 27.08 | 26.0 | 36.16 |

L—P = lidocaine-prilocaine
L = lidocaine
P = prilocaine

Statistical data

Occlusion:

Patients 1—10
L—P/Placebo: t = 6.61***
L—P/L : t = 4.40**
L—P/P : t = 2.81*

Patients 1—10
L/Placebo : t = 4.84**
P/Placebo : t = 2.92*

Non-Occlusion:

Patients 1—10
L—P/Placebo: t = 4.29**
L—P/L : t = 2.85*
L—P/P : t = 2.63*

Patients 1—10
L/Placebo : t = 1.98 n s
P/Placebo : t = 1.68 n s

* = single statistical significance and is equal to p = 0.05
** = double statistical significance and is equal to p = 0.01
*** = triple statistical significance and is equal to p = 0.001
t = the numerical value obtained when applying the Student's t-test on the data obtained.
n s = non statistical significance

Results

*Dermal analgesia*

The lidocaine-prilocaine emulsion was found to be significantly more effective than the lidocaine emulsion, the prilocaine emulsion and the placebo emulsion in producing dermal analgesia. By the Student's t-test it was found that:

| | | |
|---|---|---|
| Lidocaine-prilocaine versus placebo: | p = 0.001 | Under occlusive bandage |
| Lidocaine-prilocaine versus lidocaine: | p = 0.001 | |
| Lidocaine-prilocaine versus prilocaine: | p = 0.01 | |

| | | |
|---|---|---|
| Lidocaine-prilocaine versus placebo: | p = 0.01 | Under non-occlusive bandage |
| Lidocaine-prilocaine versus lidocaine: | p = 0.01 | |
| Lidocaine-prilocaine versus prilocaine: | p = 0.01 | |

whereby p is the statistical probability value obtained calculating the statistical significance.

The lidocaine and prilocaine emulsions were found to be insignificantly more effective than the placebo emulsion under occlusion as well as under a non-occlusive bandage. CF Table II below.

TABLE II

Pin-prick test

| Patient No | Occlusion L—P | L | P | Placebo | Non-Occlusion L—P | L | P | Placebo |
|---|---|---|---|---|---|---|---|---|
| 1 | 10/10 | 2/10 | 0/10 | 0/10 | 7/10 | 0/10 | 0/10 | 0/10 |
| 2 | 10/10 | 0/10 | 0/10 | 0/10 | 10/10 | 0/10 | 0/10 | 0/10 |
| 3 | 2/10 | 0/10 | 0/10 | 0/10 | 0/10 | 0/10 | 0/10 | 0/10 |
| 4 | 10/10 | 5/10 | 10/10 | 0/10 | 10/10 | 0/10 | 6/10 | 0/10 |
| 5 | 9/10 | 3/10 | 1/10 | 0/10 | 5/10 | 1/10 | 0/10 | 0/10 |
| 6 | 8/10 | 2/10 | 0/10 | 0/10 | 9/10 | 0/10 | 0/10 | 0/10 |
| 7 | 8/10 | 0/10 | — | 0/10 | 2/10 | 0/10 | 1/10 | 0/10 |
| 8 | 10/10 | 0/10 | 2/10 | 0/10 | 8/10 | 0/10 | 0/10 | 0/10 |
| 9 | 7/10 | 2/10 | 0/10 | 4/10 | 0/10 | 0/10 | 0/10 | 0/10 |
| 10 | 0/10 | 0/10 | 0/10 | 0/10 | 0/10 | 0/10 | 0/10 | 2/10 |
| $M_{1-10}$ | 7.40 | 1.40 | 1.44 | 0.40 | 5.10 | 0.10 | 0.70 | 0.20 |
| 11 | 10/10 | 8/10 | 7/10 | 0/10 | 5/10 | 0/10 | 2/10 | 0/10 |
| 12 | 10/10 | 6/10 | 9/10 | 0/10 | 3/10 | 0/10 | 0/10 | 0/10 |
| $M_{1-12}$ | 7.82 | 2.33 | 2.64 | 0.30 | 4.92 | 0.08 | 0.75 | 0.17 |

Statistical data

Patients 1—10 (Occlusion):
- L—P/Placebo: t = 5.78***
- L—P/L : t = 5.87***
- L—P/P : t = 4.33**

Patients 1—10 (Non-Occlusion):
- L—P/Placebo: t = 3.40**
- L—P/L : t = 3.70**
- L—P/P : t = 3.50**

Patients 1—10 (Occlusion):
- L/Placebo : t = 1.58 n s
- P/Placebo : t = 0.80 n s

Patients 1—10 (Non-Occlusion):
- L/Placebo : t = 0.42 n s
- P/Placebo : t = 0.76 n s

* = single statistical significance and is equal to p = 0.05
** = double statistical significance and is equal to p = 0.01
*** = triple statistical significance and is equal to p = 0.001
n s = non-statistical significance

All volunteers reported pain at the intradermal injection of adrenaline solution at the placebo, the lidocaine and the prilocaine areas but not at the lidocaine-prilocaine areas.

Conclusion

As evident from the results given above the antiperspirant effect is more pronounced than the analgesic effect is. This is of no disadvantage but rather the contrary, as an analgesia is not wanted using an antiperspirant day-in day-out.

The following, non-restricting Example shows a preferred embodiment of the invention. If nothing else is given parts are parts per weight and temperatures are given in degrees Celsius.

Example 1

An antiperspirant in the form of an emulsion was prepared according to the following

| | |
|---|---|
| Prilocaine, base | 5.2% by weight |
| Lidocaine, base | 4.8% by weight |
| Arlaton® 289 | 3.7% by weight |
| Carbopol® | 0.4% by weight |
| Water | 85.9% by weight |
| | 100.0 |

The local anesthetic bases are added to Arlaton® and a part of the water whereupon Carbopol® dissolved in the remaining water is added. The mixture is stirred until the emulsion is ready.

A specific advantage using the compositions according to the present invention where the active component is a local anesthetical substance of amide type is the documented low frequency of allergic complications contrary to other generally used agents.

The present compositions can also contain other active ingredients. A further aspect and preferred embodiment of the invention is thus characterized in that the antiperspirant composition of the invention further contains a compound which has an effect against sweat secretion, as e.g. an anticholinergicum or an antiadrenergicum or an agent which inhibits transportation of body liquids in the peripheral vessels.

It is also possible to introduce aluminium compounds commonly used in order to obtain combination effects.

**Claims**

1. An antiperspirant, comprising 0.5 to 100% of an actively acting component characterized in that the active component is a mixture of a first local anesthetic compound which is prilocaine, tetracaine, butanilicaine, or trimecaine in base form and a second local anesthetic compound which is benzocaine, lidocaine, bupivacaine, mepivacaine, dibucaine, etidocaine, tetracaine, butanilicaine, or trimecaine in base form.

2. Antiperspirant according to claim 1, characterized in that it is present in the form of an emulsion, whereby the amount of active component is 0.5 to 15%, the amount of emulsifier is 0.2 to 20%, the amount of thickening agent is 0.2 to 20% and the amount of water is 45 to 99.1%.

3. Antiperspirant according to any of claims 1—2, characterized in that it contains a further antiperspirant active compound, preferably an anticholinergic active compound and/or an antiadrenergic active compound.

4. Antiperspirant according to any of claims 1—2 characterized in that the active component is a mixture of prilocaine and lidocaine in the weight ratio 42:58 to 80:20.

5. Antiperspirant according to any of claims 1—2 characterized in that the active component is a mixture of prilocaine and lidocaine in the weight ratio 47:53 to 62:38.

6. Antiperspirant according to any of claims 1—2 characterized in that the active component is a mixture of prilocaine and benzocaine in the weight ratio 65:35 to 80:20.

7. Antiperspirant according to any of claims 1—2 characterized in that the active component is a mixture of prilocaine and etidocaine in the weight ratio 60:40 to 80:20.

8. Antiperspirant according to any of claims 1—2 characterized in that the active component is a mixture of prilocaine and mepivacaine in the weight ratio 85:15 to 90:10.

9. Antiperspirant according to any of claims 1—2 characterized in that the active component is a mixture of prilocaine and bupivacaine in the weight ratio 78:22 to 88:12.

## Revendications

1. Composition antiperspirante, comprenant de 0,5 à 100% d'un constituant très actif, caractérisée en ce que le constituant actif est un mélange d'un premier composé à action anesthésique locale choisi parmi la prilocaïne, la tétracaïne, la butanilicaïne ou la trimécaïne sous forme de base et d'un second composé à action anesthésique locale choisi parmi la benzocaïne, la lidocaïne, la bupivacaïne, la mépivacaïne, la dibucaïne, l'étidocaïne, la tétracaïne, la butanilicaïne ou la trimécaïne sous forme de base.

2. Composition antiperspirante selon la revendication 1, caractérisée en ce qu'elle se présente sous la forme d'une émulsion dans laquelle la quantité de constituant actif est de 0,5 à 15%, la quantité d'émulsifiant est de 0,2 à 20% et la quantité d'agent épaississant est de 0,2 à 20% et la quantité d'eau est de 45 à 99,1%.

3. Composition antiperspirante selon l'une des revendications 1 ou 2, caractérisée en ce qu'elle contient un autre composé actif antiperspirant, de préférence un composé actif anticholinergique et/ou un composé actif antiadrénergique.

4. Composition antiperspirante selon l'une des revendications 1 ou 2, caractérisée en ce que le constituant actif est un mélange de prilocaïne et de lidocaïne dans le rapport pondéral de 42:58 à 80:20.

5. Composition antiperspirante selon l'une des revendications 1 ou 2, caractérisée en ce que le constituant actif est un mélange de prilocaïne et de lidocaïne dans le rapport pondéral de 47:53 à 62:38.

6. Composition antiperspirante selon l'une des revendications 1 ou 2, caractérisée en ce que le constituant actif est un mélange de prilocaïne et de benzocaïne dans le rapport pondéral de 65:35 à 80:20.

7. Composition antiperspirante selon l'une des revendications 1 ou 2, caractérisée en ce que le constituant actif est un mélange de prilocaïne et de d'étidocaïne dans le rapport pondéral de 60:40 à 80:20.

8. Composition antiperspirante selon l'une des revendications 1 ou 2, caractérisée en ce que le constituant actif est un mélange de prilocaïne et de mépivacaïne dans le rapport pondéral de 85:15 à 90:10.

9. Composition antiperspirante selon l'une des revendications 1 ou 2, caractérisée en ce que le constituant actif est un mélange de prilocaïne et de bupivacaïne dans le rapport pondéral de 78:22 à 88:12.

## Patentansprüche

1. Antiperspirationsmittel mit 0,5 bis 100% einer aktiv wirkenden Komponente, dadurch gekennzeichnet, daß die aktive Komponente ein Gemisch einer ersten lokalanästhetischen Verbindung, die Prilocain, Tetracain, Butanilicain oder Trimecain in Basenform ist, und einer zweiten lokalanästhetischen Verbindung, die Benzocain, Lidocain, Bupivacain, Mepivacain, Dibucain, Etidocain, Tetracain, Butanilicain oder Trimecain in Basenform ist, ist.

2. Antiperspirationsmittel nach Anspruch 1, dadurch gekennzeichnet, daß es in der Form einer Emulsion vorliegt, wobei die Menge aktiver Komponente 0,5 bis 15%, die Emulgatormenge 0,2 bis 20%, die Verdickungsmittelmenge 0,2 bis 20% und die Wassermenge 45 bis 99,1% beträgt.

3. Antiperspirationsmittel nach einem der Ansprüche 1 und 2, dadurch gekennzeichnet, daß es eine weitere als Antiperspirationsmittel aktive Verbindung, vorzugsweise eine anticholinergisch aktive Verbindung und/oder eine antiadrenergisch aktive Verbindung enthält.

4. Antiperspirationsmittel nach einem der Ansprüche 1 und 2, dadurch gekennzeichnet, daß die aktive Komponente ein Gemisch von Prilocain und Lidocain im Gewichtsverhältnis 42:58 bis 80:20 ist.

5. Antiperspirationsmittel nach einem der Ansprüche 1 und 2, dadurch gekennzeichnet, daß die aktive Komponente ein Gemisch von Prilocain und Lidocain im Gewichtsverhältnis 47:53 bis 62:38 ist.

6. Antiperspirationsmittel nach einem der Ansprüche 1 und 2, dadurch gekennzeichnet, daß die aktive Komponente ein Gemisch von Prilocain und Benzocain im Gewichtsverhältnis 65:35 bis 80:20 ist.

7. Antiperspirationsmittel nach einem der Ansprüche 1 und 2, dadurch gekennzeichnet, daß die aktive Komponente ein Gemisch von Prilocain und Etidocain im Gewichtsverhältnis 60:40 bis 80:20 ist.

8. Antiperspirationsmittel nach einem der Ansprüche 1 und 2, dadurch gekennzeichnet, daß die aktive Komponente ein Gemisch von Prilocain und Mepivacain im Gewichtsverhältnis 85:15 bis 90:10 ist.

9. Antiperspirationsmittel nach einem der Ansprüche 1 und 2, dadurch gekennzeichnet, daß die aktive Komponente ein Gemisch von Prilocain und Bupivacain im Gewichtsverhältnis 78:22 bis 88:12 ist.